# EUROPEAN PATENT SPECIFICATION

(11) **EP 0 994 713 B1**
(45) Date of publication and mention of the grant of the patent: **05.11.2003**
(21) Application number: 98912450.8
(22) Date of filing: 03.03.1998
(51) Int. Cl.: A61K 31/565, A61P 9/00

(54) **USE OF A 7alpha-METHYL-17alpha-ETHYNYL-ESTRANE DERIVATIVE FOR THE TREATMENT OF ATHEROSCLEROSIS**
VERWENDUNG VON 7-APHA-METHYL-17-ALPHA-ETHYNYL-ESTRANE DERIVATEN ZUR BEHANDLUNG VON ATHEROSCLEROSE
UTILISATION D'UN DERIVE DE 7alpha-METHYLE-17alpha-ETHYNYLE-ESTRANE DANS LE TRAITEMENT DE L'ATHEROSCLEROSE

(30) Priority: 05.03.1997 EP 97200646
(43) Date of publication of application: 26.04.2000
(73) Proprietor: Akzo Nobel N.V., 6824 BM Arnhem (NL)
(72) Inventor: MEULEMAN, Dirk, Gerrit, NL-5344 HC Oss (NL); ZANDBERG, Pieter, NL-5345 RX Oss (NL)
(74) Representative: Hogenbirk, Marijke
(86) International application number: EP9801293
(87) International publication number: WO98039012

(56) References cited:
- HAENGGI ET AL.: "postmenopausal hormone replacement therapy with tibolone decreases serum lipoprotein(a)" EUR. J. CLIN. CHEM. CLIN. BIOCHEM., vol. 31, 1993, pages 645-650, XP002036506
- RYMER ET AL: "effects of tibolone on serum concentrations of lipoprotein(a) in postmenopausal women" ACTA ENDOCRINOLOGICA, vol. 128, 1993, pages 259-262, XP002036507
- KLOOSTERBOER ET AL: "long-term effect of Org OD 14 on lipid metabolism in post-menopausal women" MATURITAS, vol. 12, 1990, pages 37-42, XP002036508
- VOLPE ET AL: "benefits and risks of different hormonal replacement therapies in post-menopausal women" MATURITAS, vol. 8, no. 4, 1986, pages 327-334, XP002036515
- RIGGS: "tibolone as an alternative to estrogen for the prevention of postmenopausal osteoporosis" J. OF CLINICAL ENDOCRIN. AND METABOLISM, vol. 81, no. 7, 1996, pages 2417-2418, XP002036516 cited in the application

## Description

The present invention relates to the use of a 7α-methyl-17α-ethynyl-estrane derivative for the manufacture of a medicament for the prophylaxis and the treatment of atherosclerosis.

The development of atherosclerosis starts with the accumulation of cholesterol in lipoproteins in the vessel wall and the subsequent development of fatty streaks (probably the earliest macroscopically recognizable lesions), which appear in the intima of the arterial vessel wall as focal collections of lipid-filled macrophages ("foam cells"). This process can progress in the formation of advanced lesions; foam cell necrosis and endothelial damage can occur leading to smooth muscle cell migration and proliferation, and to the formation of extracellular matrix. Thus atherosclerosis is the result of the interaction of a number of cell types in the vessel wall, in which increased plasma cholesterol can be the driving force (Davies, M.J., and Woolf, N.; "Atherosclerosis: what is it and why does it occur"; Brit. Heart J., 1993, 69 (suppl.), S3-S11).

Coronary heart disease (CHD) is a consequence of atherosclerotic processes in the artery vessel wall. It is well known that the incidence of CHD in women in the reproductive stage of life is much lower than in men of similar age but that the risks sharply increase following the menopause.

The menopause has been associated with a large number of vasomotor, psychological and gynecological symptoms, part of which are characteristic for the perimenopausal period (climacteric). The menopause has been shown to be a risk factor for chronic diseases like osteoporosis and atherosclerosis. The sharply decreasing concentrations of estrogens, especially of estradiol (estra-1,3,5 (10)-triene-3,17-diol) and estron (3-hydroxy-estra-1,3,5 (10)-triene-17-one), in post-menopausal women have been suggested to be related to these symptoms. Estrogen replacement therapy, through which the physiologic deficit of mainly estrogen is to be corrected, is gaining increasing acceptance as a means of alleviating climacteric symptoms in peri- and post-menopausal women and preventing osteoporosis. In addition, exogenous - estrogen is reported to have a plasma cholesterol- and a LDL (low density lipoproteins)-cholesterol lowering effect and/or a plasma HDL (high density lipoproteins)-cholesterol increasing effect. These estrogen effects may be suggestive of a protective overall effect on the formation of atherosclerotic lesions.

However, clinically unopposed estrogen replacement therapy in post-menopausal women can increase the risk of endometrial hyperplasia and endometrial cancer. Therefore most therapies under study to date concern combined treatments with both an estrogen component and a progestagen component, which is added to negate the estrogen mediated risks (hormone replacement therapy). But progestagens can have adverse effects on the plasma lipoprotein concentrations and antagonize the beneficial effects of estrogen on the arterial vessel.

A synthetic steroid, 7α-methyl-17α-ethynyl-17β-hydroxy-estra-5(10)-en-3-one (Org OD-14; tibolone), produced by Organon, The Netherlands, characterized by having a mixed profile of weak estrogenic, progestogenic and androgenic properties, has been shown to be clinically as effective as estradiol valerate or conjugated equine estrogens in reducing climacteric symptoms in peri-menopausal women. Tibolone has further been shown, like the long-term administration of estrogen, to provide substantial protection against the development of osteoporosis in post-menopausal women (Hannover, N. et al., J. Clin. Endocrinology and Metabolism (1996), 81, 2419-2422).
In addition, the effect of tibolone on plasma lipoprotein concentrations has been subject of several studies. In early studies, the overall changes in total cholesterol, total glycerides or LDL and HDL cholesterol levels as a result of tibolone treatment after a 6-month period were reported to be not significant, implying that no negative side effects with respect to the early development of CHD were to be expected. Therefore, the compound was considered a useful alternative to conventional hormonal replacement therapy in post-menopausal women. (Maturitas, vol.8, no.4, 1986, pages 327-334). Studies assessing the long-term effects of tibolone treatment on lipid metabolism confirmed these findings. (see e.g. Maturitas, vol.12, no.1, 1990, pages 37-42). Other studies emphasize the negative effect of tibolone on HDL levels (a decrease, which is associated with an increased CHD risk), but describe a benificial decreasing effect found on lipoprotein(a) (Lp(a)) levels, which according to the authors may help to restore the balance of (cardiovascular) risks associated with tibolone therapy. (Acta Endocrinologica, vol. 128, 1993, pages 259-262; Eur. J. Clin. Chem. Clin. Biochem., Vol. 31, 1993, pages 645-650).
[Lp(a) is a cholesterol-rich lipoprotein which resembles LDL but is present only in trace amounts in most individuals. Those with elevated (>300 mg/l) serum Lp(a) concentrations are at high risk of CHD. Lp(a) is an independent risk marker for CHD.]
Nevertheless, the effect of long-term treatment with tibolone on HDL-cholesterol has been interpreted as less favorable with respect to its protective effect against cardiovascular disease, as compared with estrogen replacement therapy (Riggs, B.L., J. Clin. Endocrinology and Metabolism (1996), 81, 2417-2418).

Surprisingly, it has now been found that tibolone, prodrug forms thereof and certain metabolites thereof have strong anti-atherosclerotic properties. These properties are much more pronounced than those of e.g. 17β-estradiol.

The present invention therefore relates to the use of 7α-methyl-17α-ethynyl-estrane derivatives having the general formula I 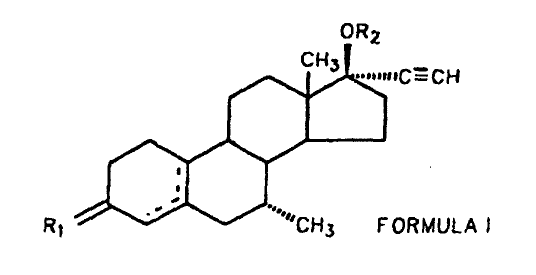 wherein
R₁ = H(OR₃) or O; R₂ = H or (C₁₋₁₈)Acyl; R₃ = H or (C₁₋₁₈)Acyl;
and the dotted line represents a double bond in the 4,5- or the 5,10-position, for the manufacture of a medicament for the prophylaxis or the treatment of atherosclerosis.

The compounds of the invention, more specifically the compounds of formula I, wherein R1 is H,OH or O, in particular those wherein R₂ = H and wherein the double bond is at the 5,10-position, and especially the compound wherein R1 is O, R2 is H and wherein the double bond is in the 5,10-position (tibolone; Org OD-14), have a very pronounced atheroprotective effect.

Thus, suitable 7α-methyl-17α-ethynyl-estrane derivatives having general formula I which can be used according to the invention are, for example, 7α-methyl-17α-ethynyl-17β-hydroxy-estra-5(10)-en-3-one (Org OD-14;tibolone). 7α-methyl-17α-ethynyl-estra-5(10)-en-3α, 17β-diol, 7α-methyl-17α-ethynyl-estra-5(10)-en-3β, 17β-diol, 7α-methyl-17α-ethynyl-17β-hydroxy-estra-4-en-3-one, and esters thereof. Preferred derivatives are tibolone and 7α-methyl-17α-ethynyl-estra-5(10)-en-3α,17β-diol. A particular preferred 7α-methyl-17α-ethynyl-estrane derivative is tibolone.

The term acyl means an acyl group derived from an organic carboxylic acid having 1-18 carbon atoms, as is also indicated by the affix (C₁₋₁₈). Examples of such carboxylic acids are formic acid, acetic acid, propionic acid, butyric acid isobutyric acid, trimethylacetic acid, valeric acid, caproic acid, capric acid, undecylenic acid, lauric acid, palmitic acid, oleic acid, phenylacetic acid, phenylpropionic acid, benzoic acid, fumaric acid, maleic acid, succinic acid and citric acid. Preferred acyl groups have 1-6 carbon atoms, and most preferred is the acetyl group.

The 7α-methyl-17α-ethynyl-estrane derivatives according to formula I are known compounds. The compounds can thus be prepared as described, for example, in US Patent 3,340,279 and in US Patent 4,701,450 for 7α-methyl-17α-ethynyl-17β-hydroxy-estra-5(10)-en-3-one (tibolone).

The use of the compounds according to the present invention does not merely lead to an absence of negative cardiovascular side effects in hormone replacement therapy as might be derived from certain prior art sources, but the compounds even have an unexpected, significant and beneficial atheroprotective effect. The 7α-methyl-17α-ethynyl-estrane derivatives according to the invention, and pharmaceutical preparations based thereon, have a beneficial effect on the cholesterol accumulation in the vessel wall, the fatty streak formation and the advanced lesion formation.

These direct effects on the vessel wall were observed in a general accepted, relevant and validated atherosclerosis model in rabbits. Contrary to expectation, in view of the weak estrogenic activity of the compounds of the invention, the remarkable and unpredicted strong atheroprotective effect of the compounds of the invention has been demonstrated down to very low doses and were much stronger in comparison with the atheroprotective effect of 17β-estradiol.

Although the compounds of invention -in contrast to estradiol- can in humans lower the plasma concentration of lipoprotein Lp(a), this can not be the explanation for the much more pronounced atheroprotective effect. In contrast to humans in rabbits no Lp(a) is present in the plasma (rabbits do not synthesize Lp(a); see Science, vol. 246, 1989, p.904-910). Therefore the strong atheroprotective effect of the compounds of this invention in (atherosclerotic) rabbits are independent of an effect on Lp(a). Consequently, when in humans a decrease in Lp(a) plays an additional role in atheroprotection, then the unexpected strong atheroprotective effect observed in rabbits might even be stronger in humans.

The 7α-methyl-17α-ethynyl-estrane derivatives of the present invention thus have a strong intrinsic atheroprotective potential and are therefore not only useful drugs in hormone replacement therapy of peri- and post-menopausal women, but they are also suitable for therapeutic use in the treatment of atherosclerosis in mammals, both male and female, of all ages. Additionally, they can also be used prophylactically to prevent atherosclerosis.

The present invention therefore provides a method of inhibiting the process of atherosclerosis comprising administering to a mammal, preferably to a human, an atheroprotective amount of a 7α-methyl-17α-ethynyl-estrane derivative having the general formula I, as previously defined.

The compounds of the present invention may be used alone or in combination with each other or with one or more other atheroprotective drugs, provided that they do not negatively interfere with each others action. A medical professional will know which drugs and combinations to choose.

The 7α-methyl-17α-ethynyl-estrane derivatives according to the invention may be administered enterally or parenterally and for humans in a daily dosage of 0.05 -10 mg, preferably 0.1-2.5 mg.

A daily dose can be administered in one or more dosage units through for example the oral, the rectal, the sublingual route, the nasal route or through the skin (for example, transdermal patches, or in the form of a cream). Preferably a single dosage unit a day is administered by the oral route. Alternatively, a controlled release preparation, releasing the daily required total dose as defined above, can be used. Controlled release preparations can be taken by the oral route, or are preferably applied in the form of a subcutaneous implant.

The pharmaceutical preparations for use according to the invention can be prepared in accordance with standard techniques such as for example are described in the standard reference, Gennaro et al. (Ed.), Remmington's Pharmaceutical Sciences, (18^{th} ed. Mack Publishing Company, 1990, e.g. Part 8: Pharmaceutical Preparations And Their Manufacture). For the purpose of making the pharmaceutical preparations according to the invention, the 7α-methyl-17α-ethynyl-estrane derivatives according to formula I, or pharmaceutically acceptable salts thereof, are mixed with or dissolved in a pharmaceutical acceptable carrier. Examples of such preparations are tablets, pills, suppositories, (micro-)capsules, powders, emulsions, creams, ointments, suspensions, solutions, implants, or sprays.

Examples of pharmaceutically acceptable carriers are: starch (for example potato or com starch), sugars (for example lactose), lubricants (for example magnesium stearate), binders (for example amylopectine or polyvinyl pyrrolidone), water, alcohol, glycerol and its derivatives, vegetable-, animal- and mineral oils and fats, fatty alcohols, silicones, polyalkylene glycols, cellulose derivatives, silica, dispersants, emulsifiers, surfactants, anti-oxidants, colorants and preservatives. In fact, any conventional pharmaceutical carrier that does not interfere with performance of the active ingredient can be used in the preparations according to the present invention.

Pharmaceutical preparations of the preferred 7α-methyl-17α-ethynyl-estrane derivative of the invention, i.e. 7α-methyl-17α-ethynyl-17β-hydroxy-estra-5(10)-en-3-one (Org OD-14; tibolone), are preferably prepared using the crystalline pure monoclinic (P2₁) form of Org OD-14, because of its improved stability, bioavailability and shelf-life. The synthesis and use in a pharmaceutical preparation of this monoclinic derivative of Org OD-14 is disclosed in European Patent No. 0,389,035B1.

The invention is illustrated by the following examples:

### General

The atheroprotective properties of 7α-methyl-17α-ethynyl-estrane derivatives according to formula I are revealed in a cholesterol fed rabbit model wherein the effects of the compounds on the atherogenesis in female ovariectomized rabbits are established. The model is considered relevant to the human atherosclerotic process, because the cellular events occurring during the development of the atherosclerotic lesions during the atherogenic diet are similar to those observed in different stages of atherosclerotic processes in coronary arteries. Rabbits, however, do not have Lp(a) in their plasma.

### Example 1

Tablets were prepared from a basic granulate containing lactose (100 mg per tablet) and dried potato starch (10 mg per tablet). The base granules were prepared by mixing the lactose with a portion of the starch. The remainder of the starch was mixed to a slurry with water and added to the mixture. The whole was granulated and dried. These base granules were mixed with ascorbyl palmitate (0.2 mg per tablet) and with either one of OD14 (2 mg or 6 mg per tablet) or 17β-estradiol (4 mg per tablet), sieved, finely mixed with magnesium stearate (0.5 mg per tablet) and then tabletted.

### Example 2.

An experiment was performed with 7 groups of sexually mature, virgin female New Zealand White rabbits (Harlan, Zeist, The Netherlands), age 7-9 months and weighing approximately 3 kg (number of rats per group: 13-14). During the acclimatization period the rabbits were fed a diet of standard commercial rabbit chow LKK20 (Hope Farms, Woerden, The Netherlands). Three weeks prior to the start of the experiment the animals were anaesthetized and underwent bilateral ovariectomy (OVX) or were sham operated. After three weeks, at the start of the experiment, the rabbits were randomized over the treatment groups. In all animals de-endothelialisation of a segment of the left carotid artery was applied by using the air-drying technique (Fishman, J.A. et al., "Endothelial regeneration in the rat carotid artery and the significance of endothelial denudation in the pathogenesis of myointimal thickening", Lab. Invest. 1975, 32, 339-351; and Lafont, A. et al., "Restenosis and experimental angioplasty: Intimal, medial and adventitial changes associated with constructive remodeling", Circulation Research, 1995, 76, 996-1002)

The animals were randomly allocated into 7 experimental groups using a randomized block design. The groups were fed an atherogenic diet (commercial rabbit chow (LKK20) enriched with 0.4 grams cholesterol, 3.75 grams coconut oil and 3.75 grams peanut oil per 100 grams). One group was fed the standard rabbit chow (LKK20). Food intake was restricted to 80 grams daily.

The treatments (Table I) were daily administered orally as a tablet, prepared as in Example 1. Groups 1, 2 and 3 were on a daily placebo treatment. Groups 4-5 were treated with 7α-methyl-17α-ethynyl-17β-hydroxy-estra-5(10)-en-3-one (OD-14; tibolone; 6 or 2 mg daily); group 6 with 17β-estradiol (4 mg daily). Group 7 was on treatment with 17β-estradiol decanoate (150 µg in 1 ml arachis oil injected subcutaneously once a week).

**Table I:**

| Design of the experiment | | | | | |
|---|---|---|---|---|---|
| Group | n | Treatment* | Dose | Diet | OVX |
| 1 | 14 | Placebo | | cholesterol | Yes |
| 2 | 13 | Placebo | | cholesterol | No |
| 3 | 13 | Placebo | | normal | Yes |
| 4 | 14 | Org OD14 | 6 mg | cholesterol | Yes |
| 5 | 13 | Org OD14 | 2 mg | cholesterol | Yes |
| 6 E2 | 14 | Estradiol | 4 mg | cholesterol | Yes |
| 7 E2D | 14 | Estradiol decanoate | 150 µg | cholesterol | Yes |

| | | | | | |
|---|---|---|---|---|---|
| *The doses were administered orally except for group 7 in which the dose was injected subcutaneously once a week. | | | | | |

During the experiment blood samples were drawn out of the central ear artery after sedation with Hypnorm (0.1 ml i.m.) (Janssen Pharmaceutics, Beerse, Belgium) before the daily treatment, at week 4,8,12,16 and 20, to monitor the plasma cholesterol levels and to measure, at week 17, plasma estradiol levels and plasma tibolone levels.
Twenty weeks after the start of the experiment animals were anaesthetized by an i.m. injection of Hypnorm (0.5 ml/kg). After blood sampling the rabbits were killed by exsanguination and the aortic arch, uterus and carotid artery removed for further analyses.

### Example 3

In addition to example 2 an additional experiment was performed with two lower doses of Org OD14 and a higher dose of estradiol decanoate. These groups together with a placebo group and a control group resulted in an experiment with 5 groups (see table II).
The procedures were identical to those in example 2.

Three weeks prior to the start of the experiment the animals were ovariectomized. At the start of the experiment the carotid artery was de-endothelialised using the air-drying technique. The normal rabbit chow was replaced by the atherogenic diet except for the control group which received during the experiment the normal rabbit chow.

**Table II:**

| Design of the experiment | | | | | |
|---|---|---|---|---|---|
| Group | n | Treatment* | Dose | Diet | OVX |
| 1 | 22 | Placebo | | cholesterol | Yes |
| 2 | 13 | Placebo | | normal | Yes |
| 3 | 13 | Org OD14 | 0.6 mg | cholesterol | Yes |
| 4 | 11 | Org OD14 | 0.15 mg | cholesterol | Yes |
| 5 E2D | 14 | Estradiol decanoate | 300 µg | cholesterol | Yes |

| | | | | | |
|---|---|---|---|---|---|
| *The doses were administered orally except for group 5 in which the dose was injected subcutaneously once a week. | | | | | |

The treatments (table II) of group 1, 2, 3 and 4 were daily administered orally as a tablet, prepared as in example 1. The doses of Org OD14 per tablet, however, were 0.6 mg and 0.15 mg respectively. Group 5 was on treatment with 17β-estradiol decanoate (300 µg in 1 ml arachis oil injected subcutaneously once a week).
Twenty weeks after the start of the experiment was ended in the same manner as described in example 2.

### EVALUATION OF ATHEROSCLEROSIS

### A: Fatty streaks

The aortic arch was dissected free, opened longitudinally and fixed in 2% paraformaldehyde. The tissue was then stained for lipids using 0.3% (w/v) Sudan Red. Colour photographs were taken of all segments. The percentage coverage of the aortic arch (Table II) with fatty streaks was assessed using image analysis (Context Vision Systems AB, Linköping, Sweden).

### B: Vessel wall cholesterol measurement

After fatty streak measurement the aortic arch was minced in a dismembrator (Mikro-Dismembrator, B. Braun, Melsungen, Germany), followed by a lipid extraction according to the method of Bligh and Dyer (Can. J. Biochem. Biophys. 1959, 37, 911-917). The total cholesterol content in the chloroform/methanol extraction solution was, after evaporation under nitrogen and dissolution in methanol, determined using enzymatic CHOD-PAP method (cat. no. 1442341, Boehringer Mannheim, Germany) and evaluated in a spectrophotometer (wavelength 500 nm). The amount of protein in the tissue was determined by the method of Lowry.

### C: Intimal thickening after de-endothelialisation

The left (airdried) carotid artery was dissected and fixed in 2% paraformaldehyde containing 6.8% glucose. The right carotid artery was used as comparison. After fixation the tissue was divided in blocks with a length of 2 mm and embedded in paraffin (Paraplast plus®, Sherwood Medical Co, St. Louis, USA) using an automated tissue processor (Hypercenter XP, Shandon).

To measure intimal thickening (using image analysis) a specific staining method and a belonging image analysis application have been developed.

Measurements were performed on 2 µm transverse sections which were treated with elastase (Serva Feinbiochemica Gmbh, Heidelberg, Germany) prior to elastin staining with Lawson solution (Boom, Meppel, the Netherlands) and light green (Sigma). Subsequently sections were airdried and mounted in Pertex (Leica Gmbh, Nussloch, Germany).

For morphological study both methylene blue/Azur II and hemotoxylin/eosin stained (2 µm) transverse sections were used. Smooth muscle cells and macrophages were detected with respectively α-actin antibodies (Sigma) and anti-macrophage antibodies (RAM11, DAKO, Glostrup, Denmark). For detection of bound antibodies goat anti-mouse ultra small gold conjugated secondary antibodies (Aurion, Wageningen, The Netherlands) and the immunogold-silver enhancement technique (SilvEnhance-LM Kit, Zymed) were used.

Images of the sections were obtained using a black and white video camera. (MX-5 , Adimec Image Systems BV, Eindhoven) mounted upon a light microscope (Axioplan, Zeiss, Jena, Germany). The video image was digitized and the intimal thickening was measured using a semi-automated image analysis application (Context Vision Systems AB, Linköping, Sweden).

Statistics: Data were expressed as mean ± S.E.M. unless otherwise specified. For testing statistical significance the Analysis of Variance (ANOVA) was used. The data were logarithmically transformed to normalize variations. A value of P<0.05 was considered to be significant.

### RESULTS (example 2, Table III)

After 20 weeks of diet and of daily treatment, necropsy was performed to determine the accumulation of cholesterol and fatty streaks in the aortic arch and the advanced lesions in the carotid artery. Moreover the weight of the uterus was determined. The results are presented in the Table III.

No significant difference in any of the variables measured were found between the ovariectomized animals (group 1) and the non-ovariectomized animals (group 2). This confirms that in the non-ovariectomized female rabbits the endogenous basal plasma estradiol levels are low.

Oral administration of 17β-estradiol (4 mg per day; group 6; E2) resulted in peak plasma estradiol levels of 238 pg/ml at 1h after administration, which was reduced to 18 pg/ml after 24 hours. Subcutaneous administered estradiol decanoate (150 µg per week; group 7; E2D) resulted in rather stable plasma estrogen levels over the day (about 60 to 70 pg/ml). The plasma levels indicate that both modes of administration of estradiol lead to effective plasma levels.
Using the human doses of estradiol and Org OD14 used for HRT treatment and corrected for the caloric intake it was expected that in the rabbit an oral dose of 4 mg estradiol is about equipotent with 6 mg Org OD14.
Measurement of the plasma levels in the rabbit showed that Org OD14 at a dose of 6 mg per day resulted in plasma concentrations of Org OD14 comparable to those obtained in women with the clinical dose of 2.5 mg.

Org OD14 (2 and 6 mg per day) increased uterus (estrogenic activity) weight to a similar extent as both estradiol treatments (E2 or E2D), confirming the equipotency of the doses used.

Despite this equipotent activity of the estrogen and Org OD14 treatments on the uterus the effect on cholesterol accumulation in the aorta and lesion formation in the aorta were different: 17β-estradiol (E2) treatment did not lead to a reduction in cholesterol accumulation, 17β-estradiol decanoate(ED2) treatment reduced cholesterol accumulation in the aortic arch with 46%, while Org OD 14 completely prevented cholesterol accumulation in the aortic arch at both concentrations studied.
17β-estradiol (E2) and 17β-estradiol decanoate (E2D) did not affect plasma cholesterol levels while Org OD14 strongly reduced the plasma cholesterol levels.
Fatty streak formation in the aortic arch was only slightly reduced by estrogen treatment while Org OD14 (nearly) completely prevented the fatty streak formation.
17β-estradiol (E2) had no effect on advanced lesion formation, following mechanical de-endothelisation, while 17β-estradiol decanoate (E2D) reduced advanced lesion formation. Histology showed that in placebo animals on a cholesterol diet the lesions consisted of smooth muscle cells and foam cells while in animals on a normal diet only smooth muscle cell were observed in the lesions. Histology of the E2D treated animals showed that the lesions still consisted of smooth muscle cells and foam cells. Org OD14 strongly inhibited the formation of advanced lesions. Lesion formation was even less than in the animals on a normal diet (control group). Histology showed that the lesions consisted only of smooth muscle cells.

### RESULTS (example 3, Table IV)

In example 2 we found that 2 and 6 mg Org OD14 in the rabbit strongly inhibited the development of atherosclerotic lesion formation. These effects were unexpectedly much more pronounced than of estradiol or estradiol decanoate while there was an equipotent estrogenic activity on the uterus.

In example 3 we tested Org OD14 at two lower doses (0.6 and 0.15 mg orally once daily) and estradiol decanoate at a higher dose (300 µg s.c. once weekly). The aim was to obtain a dose of Org OD14 which had equipotent anti-atherosclerotic effects as estradiol decanoate.
The results show that Org OD14 at 0.6 mg still nearly completely prevented _ atherosclerotic lesion formation. A two times higher dose of estradiol decanoate (compared to table III) still only partially inhibited athersclerotic lesion formation.
A dose between 0.60 mg-0.15 mg Org OD14 will show about equipotent anti-atherosclerotic effects as the 300 µg dose of estradiol decanoate. The effect on the uterus, however, was completely different. While the uterus weight increased from 0.054 to 0.132 grams (144 % increase) for Org OD14 it increased to 0.505 grams (835% increase) per 100 grams body weight for estradiol decanoate.

### CONCLUSION

The results of example 2 demonstrate that while 17β-estradiol, 17β-estradiol decanoate and Org OD14, in clinically equivalent doses, are approximately equally potent on the uterus growth in rabbits, the beneficial effects of OD14 on plasma cholesterol, cholesterol accumulation in the aortic arch and advanced lesion formation in the carotid artery, are much more pronounced than those of 17β-estradiol.
Furthermore, as demonstrated in example 3, at a dose between ten to forty times lower (0.60-0.15 mg) Org OD14 had about equipotent anti-atherosclerotic effects as a two times higher dose of estradiol decanoate (300 µg). The estrogenic effect (increase in uterus weight) for estradiol decanoate, however, was much more pronounced than for Org OD14.
These results indicate that Org OD14 has intrinsically the potential to be clinically effective for the prevention and treatment of atherosclerosis.

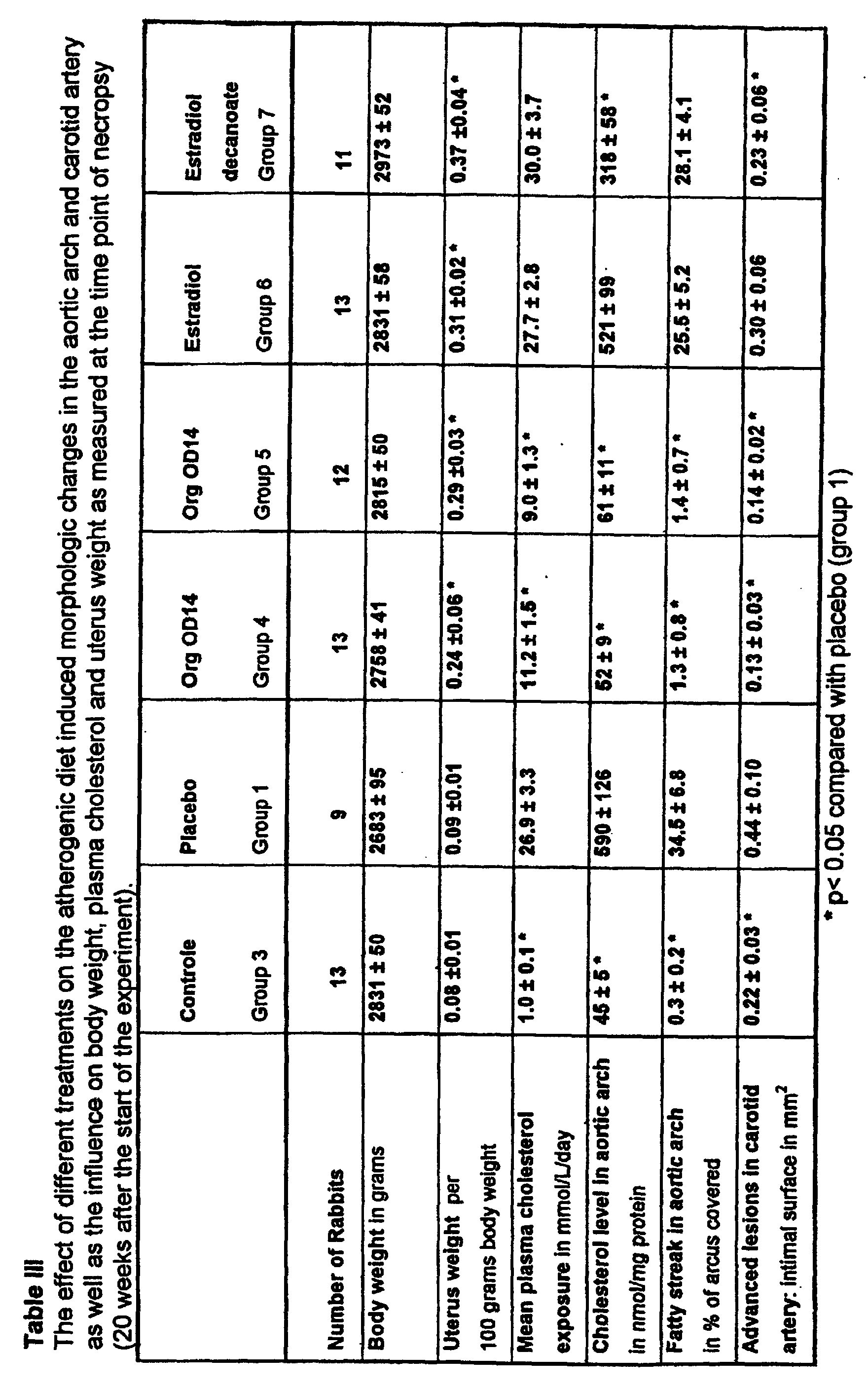

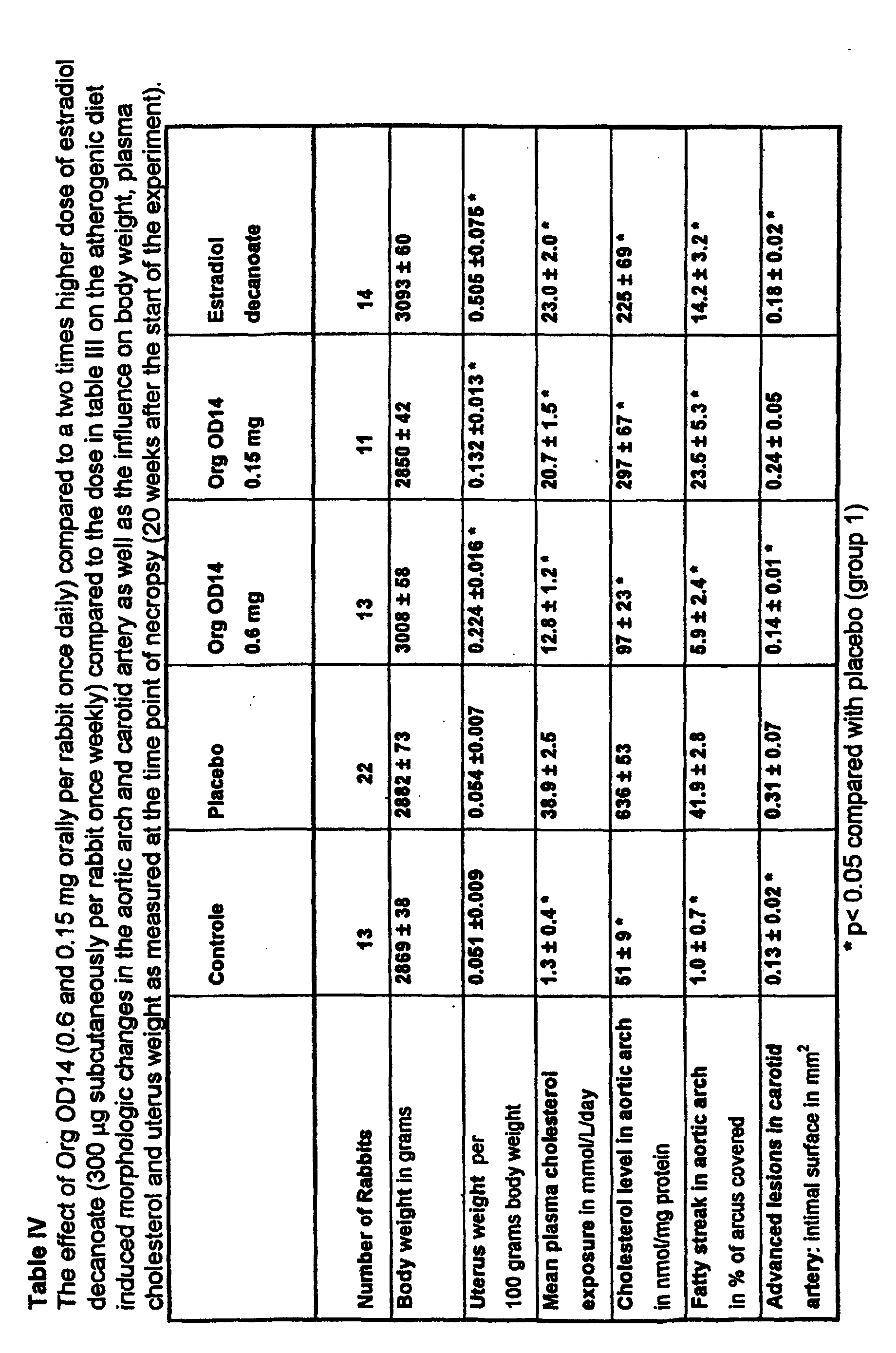

## Claims

1. Use of a 7α-methyl-17α-ethynyl-estrane derivative having the general formula I 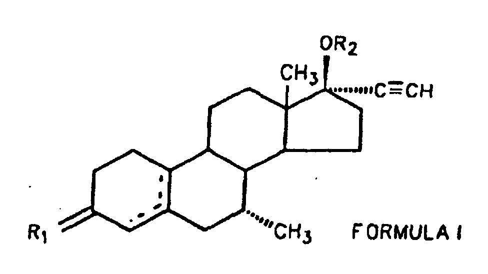 wherein
R₁ = H(OR₃)
or O;
R₂ = H or (C₁₋₁₈)Acyl;
R₃ = H or (C₁₋₁₈)Acyl;
and the dotted line represents a double bond in the 4,5- or the 5, 10-positron, for the manufacture of a medicament for the prophylaxis or the treatment of atherosclerosis.

2. Use according to claim 1, wherein R, = H,OH or O.

3. Use according to claim 1 or 2, wherein R₂ = H and the dotted line represents a double bond in the 5,10-position.

4. Use according to claim 1, wherein the 7α-methyl-17α-ethynyl-estrane derivative is 7α-methyl-17α-ethynyl-17β-hydroxy-estra-5(10)-en-3-one.

## Patentansprüche

1. Verwendung eines 7α-Methyl-17α-ethinyl-östran-Derivats der allgemeinen Formel I 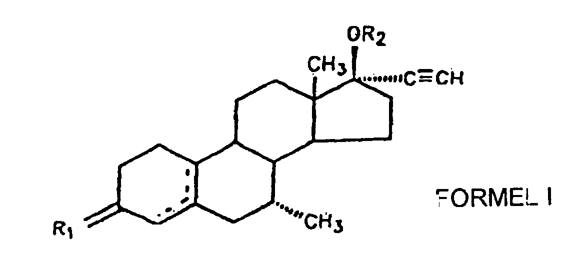 in der
R₁ = H(OR₃)
oder O ist,
R₂ = H oder (C₁-C₁₈)-Acyl ist,
R₃ = H oder (C₁-C₁₈)-Acyl ist,
und die punktierte Linie eine Doppelbindung in 4,5- oder 5,10-Stellung angibt, zur Herstellung eines Arzneimittels zur Prophylaxe oder Behandlung von Arteriosklerose.

2. Verwendung nach Anspruch 1, wobei R₁ = H, OH oder O ist.

3. Verwendung nach Anspruch 1 oder 2, wobei R₂ = H ist und die punktierte Linie eine Doppelbindung in 5,10-Stellung angibt.

4. Verwendung nach Anspruch 1, wobei das 7α-Methyl-17α-ethinyl-östran-Derivat 7α-Methyl-17α-ethinyl-17β-hydroxy-östra-5(10)-en-3-on ist.

## Revendications

1. Utilisation d'un 7α-méthyl-17α-éthynyl-estrane répondant à la formule générale I 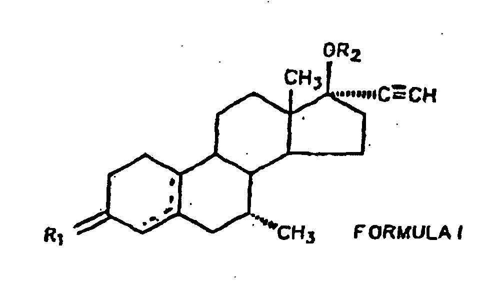 dans laquelle
R₁ = H(OR₃)
ou O ;
R₂ = H ou acyle (en C₁₋₁₈) ;
R₃ = H ou acyle (en C₁₋₁₈) ;
et la ligne pointillée représente une double liaison en positions 4, 5 ou 5,10, pour la préparation d'un médicament pour la prophylaxie ou le traitement de l'athérosclérose.

2. Utilisation selon la revendication 1, dans laquelle R₁ = H, OH ou O.

3. Utilisation selon l'une quelconque des revendications 1 ou 2, où R₂ = H et la ligne pointillée représente une double liaison en position 5, 10.

4. Utilisation selon la revendication 1, dans laquelle le dézivé de 7 α-méthyl- 17α-éthynyl-estrane est la 7α-méthyl-17α-éthynyl-17β-hydroxy-estra-5(10)-èn-3-one.
